# EUROPEAN PATENT SPECIFICATION

(11) **EP 3 433 617 B1**
(45) Date of publication and mention of the grant of the patent: **11.12.2019**
(21) Application number: 17721837.7
(22) Date of filing: 21.03.2017
(51) Int. Cl.: G01N 33/574

(54) **METHOD FOR QUANTITATIVE ASSESSMENT IN BIOLOGICAL SAMPLE OF THE PHOSPHORYLATED AND NON-PHOSPHORYLATED FORM OF RKIP**
VERFAHREN ZUR QUANTITATIVEN BEWERTUNG DER PHOSPHORYLIERTE UND NICHTPHOSPHORYLIERTER FORM VON RKIP IN EINER BIOLOGISCHEN PROBE
PROCÉDÉ D'ÉVALUATION QUANTITATIVE DANS UN ÉCHANTILLON BIOLOGIQUE DE LA FORME PHOSPHORYLÉE ET NON PHOSPHORYLÉE DE RKIP

(30) Priority: 21.03.2016 IT UA20161850
(43) Date of publication of application: 30.01.2019
(73) Proprietor: Fluidia S.r.l., 71122 Foggia (IT)
(72) Inventor: PAPALE, Massimo, 71122 Foggia (IT)
(74) Representative: Conversano, Gabriele
(86) International application number: PCT/IB2017/051628
(87) International publication number: WO 2017/163179

(56) References cited:
- EP-A1- 2 910 946
- WO-A1-00/50897
- WO-A1-2011/058136
- US-A1- 2004 018 570
- VENERA CARDILE ET AL: "Raf kinase inhibitor protein (RKIP) and phospho-RKIP expression in melanomas", ACTA HISTOCHEMICA, vol. 115, no. 8, 2013, pages 795-802, XP055327164,
- BRIANNE HILL ET AL: "Common reduction of the Raf kinase inhibitory protein in clear cell renal cell carcinoma", ONCOTARGET, vol. 5, no. 17, 2014, pages 7406-7419, XP055327032,
- AHRIM MOON ET AL: "Reduced expression of Raf-1 kinase inhibitory protein in renal cell carcinomaa: a significant prognostic marker", PATHOLOGY., vol. 44, no. 6, 2012, pages 534-539, XP055327018,
- SARA HUERTA-YEPEZ ET AL: "Expression of phosphorylated raf kinase inhibitor protein (pRKIP) is a predictor of lung cancer survival", BMC CANCER, vol. 11, no. 1, 2011, page 259, XP021102371,
- SAM CROSS-KNORR ET AL: "RKIP phosphorylation and STAT3 activation is inhibited by oxaliplatin and camptothecin and are associated with poor prognosis in stage II colon cancer patients", BMC CANCER, vol. 13, no. 1, 2013, page 463, XP021163556,
- JUNE ESCARA-WILKE ET AL: "Raf kinase inhibitor protein (RKIP) in cancer", CANCER AND METASTASIS REVIEWS, vol. 31, no. 3 - 4, 2012, pages 615-620, XP035124657,
- SIWEI LI ET AL: "Prognostic value of phosphorylated Raf kinase inhibitory protein at serine 153 and its predictive effect on the clinical response to radiotherapy in nasopharyngeal carcinoma", RADIATION ONCOLOGY, vol. 31, no. 5, 20 September 2016 (2016-09-20), page 615, XP055327166,

## Description

The present invention relates to a method for single or combined quantitative evaluation, in urines or other biological sample, of phosphorylated and not phosphorylated form of a protein called RAF kinase inhibitor (RKIP).

### Field of the invention

Improvements in genomics, proteomics and molecular pathology have produced many biomarkers candidates with a potential clinic value, which anyway are not so much used routinely mainly because of a strong separation of approach and view between academic research and biotech industries' research, which limits their collaboration.

The strong difference between the number of potential biomarkers and their developed tests that have reached the market can be appreciated by carrying out, through pubmed search engine, a comparative analysis between the biomarkers described in the main biological fluids and the tests currently available on the market. By entering keywords such biomarkers and serum more than one hundreds publications appear in pubmed, of which more than 7000 are reviews. The same occurs for urines described as ideal biological fluid for the research of pathology biomarkers of urogenital system in more than 21000 publications, of which about 2800 are reviews. The most part of serum and urine biomarkers, described in literature are of protein nature (more than 75000 original works, of which 5000 reviews for serum and about 10000 original works and 1400 reviews for urines). If one considers only the serum and urine biomarkers associated with cancer the number of original scientific works is reduced to about 30000 for serum biomarkers and about 4500 for the urine ones. Even if these numbers have to be considered approximately, since obviously for some much studied biomarkers there are hundreds of publications describing their application in various fields and for different aims, it is surprising that currently only 100 ELISA tests are available on the market, relating to as many tumor biomarkers approved for diagnostic, prognostic and/or predictive usage of various neoplasms. Recently Powers et al. have highlighted that in comparison to DNA and transcripts, proteins represent still today molecules able to describe more accurately a healthy and/or pathological phenotype of an individual since they are the final effectors which regulate cellular processes.

In particular, recent literature reports an ever more important role for Raf kinase inhibitor protein (RKIP) among tumor markers. RKIP is widely expresses in many tissues (above all testis, brain, epididymis, liver and kidneys) during the age of development. RKIP has been described in association with many tumors and has been introduced in a new class of proteins, the metastases suppressors, since its down-regulation is generally correlated with a major risk for metastasis.

RKIP reduction has been described in almost all tumors and in particular it has been correlated with middle and long term survival and with relapse appearance in prostate, colorectal, breast cancer, in melanoma, insulinoma, hepatocellular carcinoma, in anaplastic thyroid cancer and in renal cell carcinoma. Hence, RKIP is considered such a prognostic biomarker that the lower its expression levels are, the more aggressive and invasive the tumor is. RKIP can be found in not phosphorylated, free form or in a phosphorylated form which mediates its dimerization and changes its intracellular, molecular targets as well. Recently attention of many researchers has been shifting on the RKIP phosphorylated form (p-RKIP) which, in some cases, shows a prognostic capacity higher than the one of RKIP not phosphorylated form. This role has been described for lung cancer, breast cancer, melanoma and more recently for nasopharyngeal carcinoma.

Studies produced so far have concentrated on tissue evaluation of RKIP and p-RKIP expression levels by using mainly immunohistochemical techniques. Even if this technique represents one of the most diffused analysis in molecular diagnostics, it has some important limits, such for example the need to obtain tissue by means of invasive analysis as for example biopsy, and generally a semi-quantitative value due to subjective interpretation of results, determining variability between centres. Since for this technique, even if widely accepted in the clinical centres all over the world, there is still the need for standardizing the procedures used for sample preparation, analysis execution and results interpretation, it is clear that RKIP and p-RKIP quantitative dosing needs a different approach, not yet available so far.

ELISA technique (Enzyme-linked Immuno Assay) is an immunological analysis method, widely used in biochemistry to detect the presence of a substance by using one or more antibodies, to one of which an enzyme is bound: such analysis method belongs to the immunoenzymatic tests and is mainly used in immunology in order to detect and/od dose the presence of proteins, antibodies or antigens in the sample.

Like immunohistochemistry and cytofluorimetry, ELISA is able to analyze more markers with high sensitivity and specificity, at the same time. There exist multi-protein ELISA tests able to measure also various markers at the same time, an example thereof is the ELISA test which by measuring serum levels of 6 markers is able to identify ovarian cancer with 95,3% and 99,4% sensitivity and specificity, respectively. Among the advantages of this technique there are its quantitative nature, the rapidity of execution and the preferred application to biologic fluids which is associated with a less invasiveness in sampling, and as a consequence, to a greater acceptance by the patient.

Even if on the market ELISA tests exist that measure RKIP levels, they have RKIP evaluation in cellular protein extracts as main usage indication. Moreover, so far it is not available on the market a ELISA test specific for p-RKIP quantitative dosage. Finally, even if many companies provide ELISA tests useful for combined dosage of specific proteins in phosphorylated and not phosphorylated form, there is no test on the market, able to measure simultaneously RKIP/p-RKIP by this method. At the state of the art, there are known evaluation examples by means of RKIP and p-RKIP expression Western Blot analysis, which anyway have limits which make them not repeatable and/or not quantitative.

A first example is described in VENERA CARDILE et. Al: "Raf kinase inhibitor protein (RKIP) and phospho-RKIP expression in melanomas" (ACTA HISTOCHEMICA, vol. 115, no 8 - 2013 - p. 795-802). In section "western blotting" it is stated that RKIP and pRKIP expression has been evaluated by Western blot analysis of WM35 melanocytes, by a method which uses polyclonal antibodies obtained by a rabbit and which leads to not quantitative values, expressed as *arbitrary densitometric units.* In WO2011/058136 it is described a method for measuring proteins in biological sample, which uses a calibration reagent able to recognize an epitope of interest. In the described method, the calibration reagent comprises: a peptide containing a phosphorylated epitope of interest between 12 and 25 amino acids in length; a linker (peptide between 2 and 10 amino acids); a carrier protein which is bound to the array. In the method described in WO 2011/058136, to detect the standard calibration curve the calibration reagent is first bound to the various array wells with a plurality of known concentrations and then a second detectable reagent is added which is affine to the calibration reagent. Then the optical density of the signal is measured for each concentration of the calibration reagent.

At this point, to measure the presence of a protein in a biological sample one or more biological sample is bound to the array wells and an antibody is added, which is affine to the protein to be detected. Then the signal optical density is measured and compared with the optical densities measured for the standard curve.

The just described method has some technical drawbacks: in fact when the biological sample is directly added to a ELISA microtiter plate, as suggested, the presence, in sample, of a plurality of proteins (whose type and quantity are not known) determines a competition between the same for binding to the well surface. Therefore, supposing to apply this method to RKIP and p-RKIP dosage, whenever these proteins are present in the biological sample in traces with respect to other more represented proteins, they could not even bind to the well. Anyway, in particular for RKIP phosphorylated quantity (p-RKIP) which generally represents less than 1% of RKIP quantity, the well-bound quantities are so low that the method is not reproducible. Moreover, the method known at the state of the art has a limit linked to the possibility of cross-reactions. In fact, the hypothetic presence in sample of a protein other than RKIP and pRKIP, and yet containing a sequence (epitope) very similar to the one present in RKIP and p-RKIP and recognized by the antibody added to the biological sample for detection, could produce an a-specific signal not distinguishable by the signal produced by RKIP and p-RKIP. Therefore the identity of only one amino acid sequence is sufficient to produce a false positive.

Therefore, aim of the present invention is to provide a quantitative method for measuring RKIP protein phosphorylated form (p-RKIP) in a biological sample.

According to another aim, the present invention provides a quantitative method for measuring RKIP protein in its phosphorylated and not phosphorylated form, in a biological sample. According to another aim, the present invention provides a new ELISA test, specific for RKIP quantitative dosing in not phosphorylated (RKIP) and phosphorylated form (p-RKIP) in biological sample to obtain the quantitative measurement of each form and the ratio therebetween.

Yet, another aim of the invention is to provide a couple of standard recombinant peptides usable for detection of calibration curve of both RKIP and p-RKIP, by means of the bond to the same capture antibody and the same tracer antibody used for RKIP and p-RKIP detection in the biological sample to be tested, respectively, so that the method sensitivity and specificity are increased.

Yet, the present invention provides a method for RKIP and p-RKIP quantitative dosing in biological sample, in which there are practically no possibilities to obtain a-specific signals produced by proteins other than RKIP and p-RKIP. The proposed method allows also to bind selectively the proteins of interest to ELISA microtiter plate wells, upon addition of a capture antibody, so that the sample is enriched and measurements can be reproduced.

Yet according to another aim, the present invention provides a ELISA test which, by measuring RKIP and p-RKIP urine levels individually and/or in combination allows to obtain diagnosis and/or prognosis of renal cell carcinoma.

The invention realizes the prefixed aims since it is a method for RKIP protein and p-RKIP protein quantitative measuring in a biological sample, comprising the steps of:
a) coacting to two distinct supports: a1) an antibody recognizing a first amino acid sequence present in p-RKIP protein and a2) an antibody recognizing a first amino acid sequence present in RKIP protein;
b) adding a blocking solution to both the supports,
c) adding the biological sample;
d) adding, in the respective supports d1) an antibody recognizing a second amino acid sequence present in p-RKIP protein and d2) an antibody recognizing a second amino acid sequence present in RKIP protein, one of said first and second amino acid sequence being common between RKIP and p-RKIP, and the other one being a sequence containing the phosphorylation of serine 153, and so, different for p-RKIP and RKIP;
e) adding a marker able to determine a variation of the optical properties proportional to the quantity of said antibody bound to said second amino acid sequence, when in contact with said antibody recognizing said second amino acid sequence;
f) measuring an optical property of the two samples and comparing it with a calibration curve obtained by measuring the same optical property of two series of supports, in which there have been added, in the order:
   i) an antibody recognizing said first amino acid sequence present in p-RKIP protein for the first one of said series and said first amino acid sequence present in RKIP protein for the second one of said series
   ii) a blocking solution
   iii) known and increasing quantities of a recombinant hybrid standard comprising both said first and second amino acid sequences present in p-RKIP protein for the first one of said series and both said first and second amino acid sequences present in RKIP protein for the second one of said series;
   iv) antibodies recognizing specifically said second amino acid sequence present in p-RKIP or said second sequence present in RKIP, respectively;
   v) a marker able to determine a variation of the optical properties proportional to the quantity of said antibody bound to said second amino acid sequence, when in contact with said antibody recognizing said second amino acid sequence.

According to a first preferred embodiment, the method can be realized by using a ELISA microtiter plate.

In a second preferred embodiment the method can be realized by lateral flow immunochromatography assay. In this case, supports are chromatographic cartridges, the sample is added to absorbent paper to an end of the cartridge, the antibody which recognizes the first one of said amino acid sequences is coacted in a region of the chromatographic cartridge distal to the absorbent paper and the antibody which recognizes said second amino acid sequences is in a portion of the chromatographic surface in a region proximal to the absorbent paper.

In addition, preferably but not in a limiting manner, in case of using a ELISA microtiter plate, to said antibodies recognizing said second amino acid sequences (both for measuring in biological sample and for determining the calibration curve) HRP conjugated antibodies specific for said antibodies and a chromogen (TMB) as marker able to determine a variation of optical properties when in contact with said HRP antibody, can be added proportionally to HRP antibody quantity bound to said antibodies recognizing said second amino acid sequences. In this case, the optical property measured is the optical density at 450 nm.

According to another embodiment, instead of using HRP conjugated antibodies, fluorescent probes with various wavelengths among those available on the market, as for example the ones of the Alexa Fluor family, can be used as markers of antibodies recognizing the second amino acid sequence. In that case, the signal detection is obtained by means of a fluorescence reader, and the modified optical property is the fluorescence at a determined wavelength.

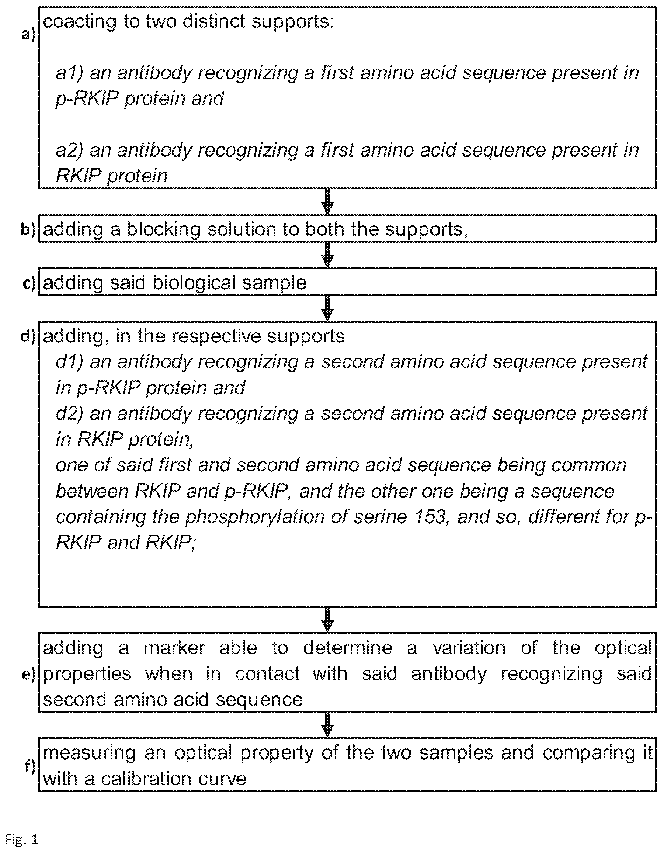

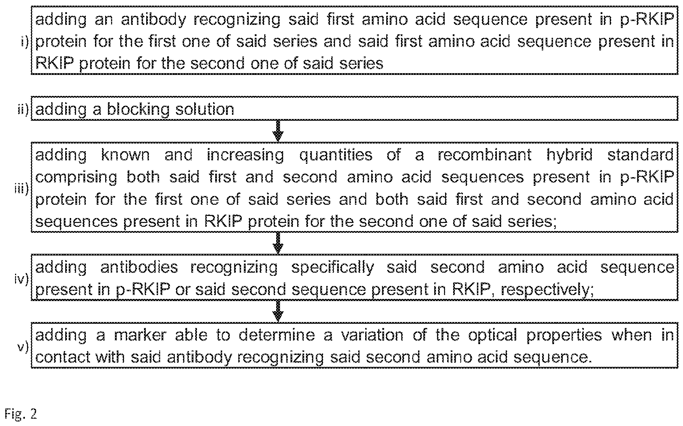

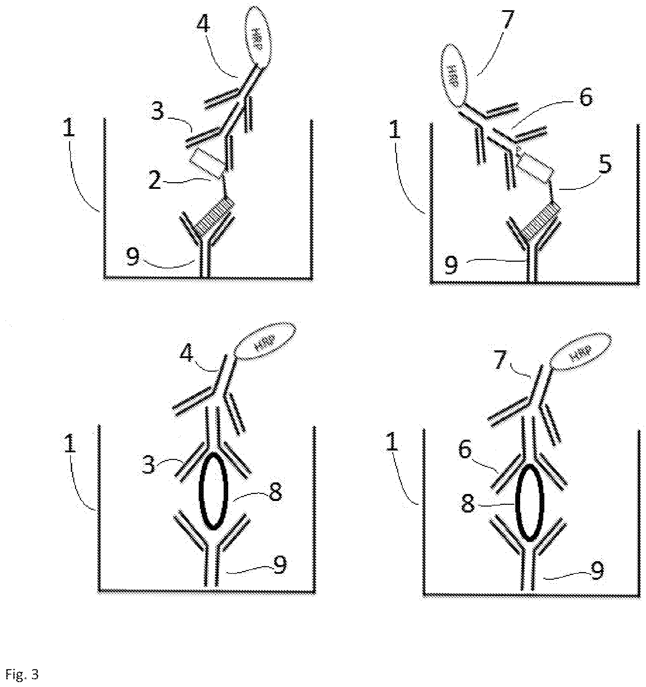

These and other advantages will be clear from the detailed description of the invention, which refers to figures 1 to 3.

A first embodiment will be now described with reference to the test realization with a ELISA microtiter plate. It is to be repeated that, without departing from the scope of the present invention, the test can be carried out by using different supports, such for example lateral flow immunochromatography (Sajida M et al., Journal of Saudi Chemical Society 2015; 19 (6) 689-705) or a microfluidic device (Sardesai NP, et al. Anal Bioanal Chem. 013 April; 405 (11): 3831-3838). Firstly it is to be stated also that the biological sample which is referred to in the following is preferably urine, but it can be any other biological fluid (as for example serum, plasma, saliva, brochoalveolar fluid, cerebrospinal fluid, stool, semen) or other biological sample (as for example cellular or tissue protein extract or cellular culture medium) where the analyte of interest is contained.

Yet another preamble valid for the various following embodiments is linked to the approach used for signal detection. In the preferred embodiment it is considered an antibody conjugated with horseradish peroxidase (HRP) and a chromogenic method based on the addition of 3,3'-5,5'-tetramethylbenzidine (TMB) substrate for detection. It is to be intended that detection could be carried out also by chemiluminescence (by using for example a luminol/H2O2/enhancer system) or by chemifluorescence (by conjugating the antibody with substrates as for example fluorescein, rhodamine, Texas red) or could be based also on the usage of interferometric sensors, as for example those described by Duval et al (Silicon Photonic Based Biosensors: The Future of Lab-on-a-Chip Diagnostic devices; IEEE PHOTONICS SOCIETY NEWLETTER, August 2012) without departing from the scope of the present invention.

Similarly the detailed experimental procedures for practical implementation of the embodiments described could be defined according to the standards of good technique, without departing from the aims of the present invention. Only as a way of example ELISA microtiter plates pre-coacted with protein A or G could be used to bind the capture antibody more stably.

### Measuring RKIP and p-RKIP quantity in the biological sample

According to a preferred embodiment, RKIP and p-RKIP quantitative dosing in the biological sample to be tested uses an antibody, in the following defined capture antibody (9) that recognizes an epitope present in the RKIP/p-RKIP shared region, having preferably, but not in a limiting manner, the amino acid sequence reported in the following (Id1 in the appended sequence listing):
DPDAPSRKDPKYRE

The capture antibody (9) is coacted to wells (1) of ELISA microtiter plate, where then the blocking solution is added which is made up of BSA, milk or fetal bovine serum in an optimal concentration between 1 and 5%, which serves to saturate the binding sites and not to allow the adhesion of analytes other than the ones recognized by the capture antibody.

In the following, the biological sample is introduced to obtain the analyte capturing before dosing. It is to be specified in fact that having previously coacted the capture antibody to the wells of ELISA microtitle plate, only proteins present in the biological sample and recognized by the capture antibody can be bound. Then, they are only RKIP and p-RKIP, or proteins containing the same amino acid sequence common to RKIP and p-RKIP. Similarly, when, after the biological sample, the tracer antibodies specific for RKIP and p-RKIP are added (see following section), they bind to RKIP or p-RKIP peculiar region. Therefore, it is absolutely unlikely that a protein other than those of interest can produce a signal, since it should contain both the exact sequence recognized by the capture antibody and the exact sequence recognized by the tracer antibody, and the probabilities this occurs for a protein other than RKIP and p-RKIP are nearly null.

Then to the biological sample are added firstly tracer antibodies specific for RKIP (3) and p-RKIP (6) respectively, and in the following, HRP conjugated antibodies specific for RKIP (4) and p-RKIP (7).

The tracer antibodies are antibodies which recognize specifically a sequence present in not phosphorylated form (tracer antibody specific for RKIP) or phosphorylated form (tracer antibody specific for p-RKIP) of protein.

HRP-conjugated antibodies bind specifically to tracer antibodies, and proportionally to the bound tracer antibody quantity they modify the absorbance (optical density) measured inside the well by means of a colorimetric reaction.

After addition of HRP conjugated antibodies, a chromogen (3,3',5,5'-tetramethylbenzidine-TMB) is added, which acts as electron donor for hydrogen dioxide reduction to water by means of an enzyme with peroxidase action as horseradish peroxidase (HRP). Therefore, the chromogen gets a blue coloration, whose intensity is proportional to the quantity of HRP antibody present in each well, which, after the reaction stop, obtained by the addition of an acid (sulphuric acid or hydrochloric acid) turns to yellow and can be read at 450 nm absorbance.

The comparison of optical density of sample with the one of the wells used for determining the calibration curve (see dedicated section) allows to determine the quantity of RKIP and p-RKIP protein present in sample.

### Detection of calibration curve

In the following it is described the procedure to be followed for detecting the calibration curve to be used for determining RKIP and p-RKIP quantitative presence.

Before the description, it is to be given the definition of recombinant hybrid peptide (or standard): a recombinant hybrid peptide for a protein of interest is a peptide which has:
i) a kept sequence of the protein of interest which can be bound to a capture antibody specific for such sequence;
ii) a second sequence of the protein of interest, which can contain specific post-translational modifications such as phosphorylation, acetylation, glycosylation, and which can be bound to second antibody (tracer antibody) able to recognize selectively the presence or not of modification.

In the case of the method according to the present invention, tracer antibodies will be specific for RKIP sequence with or without phosphorylation on serine 153 of RKIP peptide chain.

The calibration curve is obtained by using two recombinant hybrid standards whose sequence is reported in the following:
RKIP (2) recombinant hybrid standard: DPDAPSRKDPKYRE-(linker)-GDHRGKFKVASFRKKYELRA
p-RKIP (5) recombinant hybrid standard: DPDAPSRKDPKYRE-(linker)-GDHRGKFKVApSFRKKYELRA

With reference to the appended sequence listing, RKIP hybrid recombinant standard has sequences: Id2 - linker - Id3 while p-RKIP hybrid recombinant standard has sequences: Id4 - linker - Id5.

Hence, each one of the two hybrid peptides has:
- a common region (Id2 = Id4), which can be bound to the capture antibody (9);
- a linker
- a peculiar region (Id3 and Id5) which can be bound to RKIP specific tracer antibody in a case and to p-RKIP specific tracer antibody in the other case.

For detecting the calibration curve, capture antibody (9) is coacted in two series of wells (1) of ELISA microtiter plate and in the following the blocking is carried out with BSA, milk or fetal bovine serum of the well surface binding sites. Then ever increasing quantities of RKIP recombinant hybrid standard (in one of the two series of wells) or p-RKIP recombinant hybrid standard (in the other one) are added.

The capture antibody (9) will bind both the recombinant standards by means of the common region (DPDAPSRKDPKYRE).

The following addition of the two tracer antibodies, one specific for RKIP and the other specific for p-RKIP will allow to bound the peculiar regions of RKIP recombinant hybrid standard (GDHRGKFKVASFRKKYELRA - Id3 in the sequence listing) and of p-RKIP recombinant hybrid standard (GDHRGKFKVApSFRKKYELRA - Id5 in the sequence listing).

Finally, HRP-conjugated antibodies and chromogen addition will allow to measure optical density corresponding to recombinant standard quantity added in each well, thus defining standard calibration curves, in which a specific optical density is associated with each known concentration of recombinant standard.

After binding sites blocking, the biological sample to be tested (8) is added in different wells, coacted with antibody (9). In these wells, after addition of tracers antibodies for RKIP (3) and p-RKIP (6), HRP-conjugated antibodies that recognize for RKIP tracer (4) and p-RKIP tracer (7) and chromogen, respectively, the optical density will be measured, and RKIP and p-RKIP concentration will be defined by comparing the optical densities measured with standard curves ones.

It is to be noted that, without departing from the field of the invention, the method described can provide the inversion of antibodies used such that the two tracer antibodies could be coacted to the wells, thus becoming capture and the capture antibody could be used for detection, thus becoming the tracer.

Obviously it will be needed to use suitable HRP conjugated antibodies.

Independently of the type of realization, the test reaches the aim of providing RKIP and p-RKIP quantitative determination. At the best knowledge of today inventors, so far there is neither an immunoenzymatic test measuring p-RKIP quantitatively, nor a test measuring RKIP/p-RKIP levels at the same time.

In a preferred embodiment, the application of this test to urine sample can allow the early diagnosis and prognosis of renal cell carcinoma (RCC), thus defining a diagnostic parameter based on p-RKIP concentration inside the biological sample and a prognostic one based on RKIP levels.

This aspect results very important if it is considered that RCC diagnosis is often a casual event and so far it can be ascertained only by carrying out renal biopsy. The proposed invention can allow RCC screening simply, rapidly and little invasively thus allowing a significant widening of RCC diagnosis in the first disease development stages with consequent improvement in prognosis and patient life expectancy. Yet more important, in economics and prevention terms, is the test application to specific high risk patient classes such for example obese people, people with hypertension and patients with terminal renal disease, who undergo renal ultrasound only occasionally to individuate the disease early, while by means of RKIP/p-RKIp test, they could obtain rapidly and not invasively indications about the risk of developing the renal cell carcinoma, and in case of positivity, about the prognosis which is the more favourable the earlier the diagnosis is. Only considering obese people and people with hypertension, the population potentially apt for screening would be more than a billion individuals all over the world. In addition, the impact the present invention could have on the cancer diagnosis is shown by recent literature which has been highlighting an ever more important role of RKIP and p-RKIP in the genesis and progression of various cancers, among which the lung cancer (more than 1.8 million cases annually in the world), the breast cancer (more than 1.6 million cases) and colorectal cancer (almost 1.4 million cases).

The object of the present invention can be used for determining RKIP and p-RKIP intracellular expression in any type of cell and tissue. In this case, the only variation would be the addition in wells (1) of ELISA microtiter plate, as biological sample, of cellular protein extract in which RKIP and p-RKIP protein is to be determined. Hence, the test could be widely used by researcher teams committed in the characterization of RKIP and p-RKIP functional role in various pathologies, since it is a simple, rapid and quantitative method.

In particular, the present invention provides a diagnostic method for diagnosis of clear cell renal cell carcinoma based on quantitative measurement in a urine sample of p-RKIP protein and a method for prognosis of clear cell renal cell carcinoma based on quantitative measurement in a urine sample of RKIP protein. Both these measurements can be conveniently carried out according to any one of the just described modes.

The diagnostic method for clear cell renal cell carcinoma comprises the steps of:
- measuring p-RKIP quantity in a urine sample;
- in case of p-RKIP absence in the urine sample, diagnosing a clear cell renal cell carcinoma.

In case of p-RKIP presence in the urine sample, the diagnosis is negative (there is no renal carcinoma).

It is to be précised that here for p-RKIP absence in the sample is intended the absence of a measurable signal (optical density measured in sample comparable with the one obtained in absence of biological sample) in the test working conditions with the method according to the present invention, by using capture antibody + tracer antibody + HRP conjugated antibody + TMB.

This does not exclude that by using a detection system able to amplify the signal (ex. streptavidin-biotin; fluorescence etc.) it is possible to detect p-RKIP presence also in urine sample of patients with clear cell renal cell carcinoma. The difference between the minimal concentration detectable without amplifying the signal and the minimal concentration detectable with the method according to the present invention is anyway such high that p-RKIP concentration in urines of patient can be considered equal to zero if no signal is detected with the method according to the invention.

The prognosis method to anticipate the development of a clear cell renal cell carcinoma comprises the following steps of:
- measuring RKIP quantity in a urine sample;
- if RKIP quantity is higher than a threshold value, a favourable prognosis is deduced;
- if measured RKIP quantity is lower than a threshold value, a not favourable prognosis is deduced.

Concerning the exact definition of the threshold value to be used as cut-off, it is to be précised that so far it is not possible to define a threshold value for RKIP content in urine sample, since thousand measurements would be needed to define it exactly. Anyway, it is certain that this threshold value can be defined, since the urine sample reflects generally what happens in renal tissue. This was confirmed in a work, just accepted for publication on Oncotarget journal (Urinary RKIP/p-RKIP is a potential diagnostic and prognostic marker of clear cell Renal Cell Carcinoma" Papale et. Al, Oncotarget 2017) which has shown the direct correlation between tissue expression levels and RKIP and pRKIP urine excretion in patients with clear cell renal cell carcinoma.

Hill B et al (Oncotarget. 2014 Sep 15;5(17):7406-19, "Common reduction of the Raf kinase inhibitory protein in clear cell renal cell carcinoma") have shown that a linear correlation exists between RKIP levels and renal carcinoma progression. By means of a Tissue MicroArray analysis (TMA), carried out on more than 600 tissues of patients with clear cell renal cell carcinoma, they have shown that an H-score (parameter commonly used for analysis of data produced by TMA experiments) higher than 260 identifies the normal tissue, a 220 score identifies RCC presence, a 204 score identifies primary metastatic RCC presence and a 181 score the appearance of metastases in long term. Moreover, patients with H-score > 204 had a generally favourable prognosis while those with H-score < 204 had a not favourable prognosis (death in 50% of cases). Always using a TMA analysis, Papale et al. have evaluated RKIP and p-RKIP levels in renal tissue of 40 patients with clear cell renal cell carcinoma (ccRCC), 19 patients with chronic kidney disease (CKD) and 5 apparently healthy tissues by giving a score = 0 for absence of signal, 1 for low signal, 2 for middle signal and 3 for the high one. With this approach all the normal tissues had score 3 for RKIP, CKD tissues a score between 2 and 3 while only 8/40 ccRCCC had a score 1-2 (middle-low) while in the others the protein resulted not detectable. The same goes for p-RKIP which resulted very expressed in normal tissue, reduced in CKD and totally absent in all ccRCC tissues independently of the grade, presence of visceral metastases, size of tumor and lymph node involvement.

As yet said, the urine sample reflects generally what can be found also in renal tissue, anyway the current measuring techniques have not allowed to diffuse RKIP and p-RKIP quantitative measurement in urine sample. This is made possible instead by the method according to the present invention. At the state of the art, instead, the quantitative datum can be obtained only by immunoblotting, a semi-quantitative technique whose limits of difficulty and reproducibility are well known.

The object of the present invention introduces some technical innovations with respect to ELISA assays, currently available on the market, for phosphoprotein analysis, anyway other than pRKIP. In fact, even if the main Biotech companies (Thermofisher, SigmaAldrich, R&D systems, Raybiotech) have recently launched on the market ELISA tests specific for phosphoprotein dosing, in the most cases it is not provided the usage of a reference standard, thus limiting the analysis to the simultaneous dosage of the total and phosphorylated form of the same protein which is carried out by charging the well with the same volume of cellular extract for the various conditions to be tested. Even if this approach can be useful to understand the phosphorylated target protein quantity in a determined context, it is anyway not suitable for carrying out the biomarker quantitative dosage.

However, there are companies that use a reference standard: in the most cases the standard is made up of cellular extracts stimulated with specific growth factors to obtain phosphorylation of target protein. Serial dilutions of stimulated cellular extract are used to create a standard reference curve which anyway does not allow the absolute quantification of protein and its phosphorylated form.

Another advantage of the just described solution is highlighted in that now only few companies, among which NOVEX, have developed ELISA tests which use the relative recombinant protein as standard, but however for a limited number of phospho-proteins (about 32). An example is provided by the Phospho-ELISA kit TAU [pS199] test (Novex-Thermofisher, catalogue number KHB7041).

This aspect is very important because it highlights how not for all proteins exist standards suitable to make the test quantitative. In fact, not all facilities which produce recombinants are able to provide both the native and phosphorylated form. Moreover, it is important to underline an economic advantage of the proposed solution: the synthesis of recombinant protein is very much longer and more expensive than the synthesis of hybrid peptide as proposed in the present invention (about 7000 Euro for synthesis in E. Coli of native protein and, whenever possible, of the phosphorylated form of the protein of interest with respect to about 1500 Euro to obtain the synthesis of about 5 mg hybrid peptide and about 5 mg p-RKIP hybrid peptide). Therefore, with equal performance, the approach proposed seems to be more convenient and more efficient than the current solutions.

## Claims

1. Method for RKIP protein and p-RKIP protein quantitative measuring in a biological sample, comprising the steps of:
a) coacting to two distinct supports:
a1) an antibody recognizing a first amino acid sequence present in p-RKIP protein and
a2) an antibody recognizing a first amino acid sequence present in RKIP protein;
b) adding a blocking solution to both the supports,
c) adding said biological sample;
d) adding, in the respective supports
d1) an antibody recognizing a second amino acid sequence present in p-RKIP protein and
d2) an antibody recognizing a second amino acid sequence present in RKIP protein;
one of said first and second amino acid sequence being common between RKIP and p-RKIP, and the other one being a sequence containing the phosphorylation of serine 153, and so, different for p-RKIP and RKIP;
e) adding a marker able to determine a variation of the optical properties proportional to the quantity of said antibody bound to said second amino acid sequence, when in contact with said antibody recognizing said second amino acid sequence;
f) measuring an optical property of the two samples and comparing it with a calibration curve obtained by measuring the same optical property of two series of supports, in which there have been added, in the order:
i) an antibody recognizing said first amino acid sequence present in p-RKIP protein for the first one of said series and said first amino acid sequence present in RKIP protein for the second one of said series
ii) a blocking solution
iii) known and increasing quantities of a recombinant hybrid standard comprising both said first and second amino acid sequences present in p-RKIP protein for the first one of said series and both said first and second amino acid sequences present in RKIP protein for the second one of said series;
iv) antibodies recognizing specifically said second amino acid sequence present in p-RKIP or said second sequence present in RKIP, respectively;
v) a marker able to determine a variation of the optical properties proportional to the quantity of said antibody bound to said second amino acid sequence, when in contact with said antibody recognizing said second amino acid sequence.

2. Method according to claim 1, **characterized in that** said recombinant hybrid standard which comprises both said first and second amino acid sequences present in p-RKIP protein has the amino acid sequence reported in the following:
DPDAPSRKDPKYRE-(linker)-GDHRGKFKVApSFRKKYELRA

3. Method according to claim. 1 or 2, **characterized in that** said recombinant hybrid standard which comprises both said first and second amino acid sequences present in RKIP protein has the amino acid sequence reported in the following:
DPDAPSRKDPKYRE-(linker)-GDHRGKFKVASFRKKYELRA

4. Method according to claim 3, **characterized in that** both said first sequence present in RKIP and said first sequence present in p-RKIP are equal to the sequence reported in the following:
DPDAPSRKDPKYRE.

5. Method according to claim 4, **characterized in that** said second sequence present in RKIP is the following:
GDHRGKFKVASFRKKYELRA
while said second sequence present in p-RKIP is the following:
GDHRGKFKVApSFRKKYELRA

6. Method according to any one of the preceding claims, **characterized in that** said supports are wells of a ELISA microtiter plate, **in that** after point d) it is introduced the step of:
d3) adding in the respective supports HRP-conjugated antibodies specific for said antibodies which recognize said second amino acid sequences,
and after point iv) to said series of wells HRP-conjugated antibodies specific for antibodies which recognize said second amino acid sequences are added,
and **in that** said marker able to determine a variation of optical properties when in contact with said antibody recognizing said second amino acid sequence is TMB and said optical property is optical density.

7. Method according to any one of the proceding claims, **characterized in that** said supports are chromatographic cartridges,
**in that** said biologic sample is added to an end of the cartridge,
**in that** said antibodies which recognize said first amino acid sepuences are coacted in a region of said cartridge distal to the absorbent paper,
and **in that** said antibodies which recognize said second amino acid sequences are adhered to the chromatographic surface of said cartridge in a region proximal to the absorbent paper,
and **in that** said marker able to determine a variation of optical properties when in contact with said antibody recognizing said second amino acid sequence is a fluorescent probe and said optical property is fluorescence at a specific wavelength.

8. Method according to any one of the preceding claims, **characterized in that** said biological sample si one among urine, serum, plasma, saliva, brochoalveolar fluid, cerebrospinal fluid, stool, semen, cellular or tissue protein extract or cellular culture medium.

9. Diagnostic method for diagnosis of clear cell renal cell carcinoma, comprising the steps of:
- measuring p-RKIP quantity in a urine sample with the method according to any one of the preceding claims;
- in case of p-RKIP absence in the urine sample, diagnosing a clear cell renal cell carcinoma.

10. Prognosis method to anticipate the development of a clear cell renal cell carcinoma, comprising the following steps of:
- measuring RKIP quantity in a urine sample with the method according to any one of claims 1 to 6;
- if RKIP quantity is higher than a threshold value, a favourable pronosis is deduced;
- if measured RKIP quantity is lower than a threshold value, a not favourable prognosis is deduced.

## Patentansprüche

1. Verfahren für eine quantitative Messung von RKIP Protein und p-RKIP Protein in einer biologischen Probe, umfassend die Schritte:
a) Zusammenwirken mit zwei unterschiedlichen Trägern:
a1) einem Antikörper, der eine erste in der p-RKIP-Protein vorhandene Aminosäure-Sequenz erkennt und
a2) einem Antikörper, der eine erste in der RKIP-Protein vorhandene Aminosäure-Sequenz erkennt;
b) Hinzufügen einer Blockierungslösung zu beiden Trägern,
c) Hinzufügen der genannten biologischen Probe;
d) Hinzufügen in die jeweiligen Träger
d1) eines Antikörpers, der eine zweite im p-RKIP-Protein vorhandene Aminosäure-Sequenz erkennt, und
d2) eines Antikörpers, der eine zweite im RKIP-Protein vorhandene Aminosäure-Sequenz erkennt;
wobei eine der ersten und zweiten Aminosäure-Sequenzen ist gemeinsam für RKIP und p-RKIP, und die andere ist eine Sequenz, die die Phosphorylierung von Serin 153 enthält und sich daher für p-RKIP und RKIP unterscheidet;
e) Hinzufügen eines Markers, der in der Lage ist, eine Variation der optischen Eigenschaften zu bestimmen, die proportional zur Menge des genannten an die zweite Aminosäure-Sequenz gebundenen Antikörpers ist, wenn er mit dem die genannte zweite Aminosäure-Sequenz erkennenden Antikörper in Kontakt steht;
f) Messen einer optischen Eigenschaft der beiden Proben und Vergleichen derselben mit einer Kalibrierungskurve, die durch Messen der gleichen optischen Eigenschaft von zwei Reihen von Trägern erhalten wurde, zu denen in der Reihenfolge hinzugefügt wurden:
i) ein Antikörper, der die erste im p-RKIP-Protein vorhandene Aminosäure-Sequenz für die erste der genannten Reihen und die erste im RKIP-Protein vorhandene Aminosäure-Sequenz für die zweite der genannten Reihe, erkennt
ii) eine Blockierungslösung
iii) bekannte und zunehmende Mengen eines rekombinanten Hybridstandards, umfassend sowohl die erste als auch die zweite Aminosäure-Sequenz, die in p-RKIP-Protein für die erste der genannten Reihe vorhanden sind, und sowohl die erste als auch die zweite Aminosäure-Sequenz, die in RKIP-Protein für die zweite der genannten Reihen, vorhanden sind;
iv) Antikörper, die spezifisch die in p-RKIP vorhandene zweite Aminosäure-Sequenz oder die in RKIP-Protein vorhandene zweite Aminosäure-Sequenz erkennen;
v) einen Marker, der in der Lage ist, eine Variation der optischen Eigenschaften zu bestimmen, die proportional zur Menge des an die zweite Aminosäure-Sequenz gebundenen Antikörpers ist, wenn er mit dem die zweite Aminosäure-Sequenz erkennenden Antikörper in Kontakt steht.

2. Verfahren nach Anspruch 1, **dadurch gekennzeichnet, dass** der genannte rekombinante Hybridstandard, der sowohl die erste als auch die zweite im p-RKIP-Protein vorhandene Aminosäure-Sequenz umfasst, die im Folgenden angegebene Aminosäure-Sequenz aufweist:
DPDAPSRKDPKYRE- (linker) -GDHRGKFKVApSFRKKYELRA

3. Verfahren nach Anspruch 1 oder 2, **dadurch gekennzeichnet, dass** der genannte rekombinante Hybridstandard, der sowohl die erste als auch die zweite im RKIP- Protein vorhandene Aminosäure-Sequenz umfasst, die im Folgenden angegebene Aminosäure-Sequenz aufweist:
DPDAPSRKDPKYRE- (linker) -GDHRGKFKVASFRKKYELRA

4. Verfahren nach Anspruch 3, **dadurch gekennzeichnet** sowohl die erste Sequenz, die in RKIP vorhanden ist als die erste Sequenz, die in p-RKIP vorhanden ist, zu der im folgenden genannten Sequenz gleich sind:
DPDAPSRKDPKYRE.

5. Verfahren nach Anspruch 4, **dadurch gekennzeichnet, dass** die in RKIP vorhandene zweite Sequenz die folgende ist:
GDHRGKFKVASFRKKYELRA
während die zweite in p-RKIP vorhandene Sequenz die folgende ist:
GDHRGKFKVApSFRKKYELRA

6. Verfahren nach einem der vorhergehenden Ansprüche, **dadurch gekennzeichnet, dass** die genannten Träger Vertiefungen einer ELISA-Mikrotiter-Platte sind, wobei nach Punkt d) der Schritt eingeführt wird: d3) Hinzufügen in den entsprechenden Trägern von HRPkonjugierten Antikörpern, die für die Antikörper spezifisch sind, die die zweiten Aminosäure-Sequenzen erkennen, und nach Punkt iv) zu der genannten Reihe von Vertiefungen HRP-konjugierte Antikörper hinzugefügt werden, die für Antikörper spezifisch sind, die die zweiten Aminosäure-Sequenzen erkennen, und dadurch, dass der genannte Marker in der Lage ist, eine Variation der optischen Eigenschaften zu bestimmen, wenn er in Kontakt mit dem genannten Antikörper steht, der erkennt, dass die zweite Aminosäure-Sequenz ein TMB ist und die genannte optische Eigenschaft die optische Dichte ist.

7. Verfahren nach einem der vorhergehenden Ansprüche, **dadurch gekennzeichnet, dass** die genannten Träger chromatographische Kartuschen sind,
wobei die genannte biologische Probe an einem Ende der Kartusche hinzugefügt wird,
wobei die genannten Antikörper, die die ersten Aminosäure-Sequenzen erkennen, in einem Bereich der genannten Kartusche, der vom absorbierenden Papier distal ist, zusammenwirken,
und wobei die genannten Antikörper, die die genannten zweiten Aminosäure-Sequenzen erkennen, an die chromatographische Oberfläche der genannten Kartusche in einem Bereich anhaften, der proximal zu dem absorbierenden Papier ist,
und wobei der genannte Marker, der in der Lage ist, eine Variation der optischen Eigenschaften zu bestimmen, wenn er in Kontakt mit dem Antikörper steht, der die zweite Aminosäure-Sequenz erkennt, eine fluoreszierende Probe ist und die optische Eigenschaft die Fluoreszenz bei einer spezifischen Wellenlänge ist.

8. Verfahren nach einem der vorhergehenden Ansprüche, **dadurch gekennzeichnet, dass** die genannte biologische Probe eine Probe aus Urin, Serum, Plasma, Speichel, Bronchoalveolar-Flüssigkeit, Cerebrospinalflüssigkeit, Kot, Sperma, ein Zell-oder Gewebeproteinextrakt oder ein Zellkulturmedium ist.

9. Diagnoseverfahren zur Diagnose eines klarzelligen Nierenzellkarzinoms, umfassend die Schritte:
- Messen der p-RKIP-Menge in einer Urinprobe mit dem Verfahren nach einem der vorhergehenden Ansprüche;
- bei Abwesenheit von p-RKIP in der Urinprobe, eine Diagnose eines klarzelligen Nierenzellkarzinoms durchführen.

10. Prognose-Verfahren zur Antizipation der Entwicklung eines klarzelligen Nierenzellkarzinoms, umfassend die folgenden Schritte:
- Messen der RKIP-Menge in einer Urinprobe mit dem Verfahren nach einem der Ansprüche 1 bis 6;
- wenn die RKIP-Menge höher als ein Schwellenwert ist, wird eine günstige Prognose abgeleitet;
- wenn die gemessene RKIP-Menge unter einem Schwellenwert ist, wird eine ungünstige Prognose abgeleitet.

## Revendications

1. Méthode pour une mesure quantitative d'une protéine RKIP et d'une protéine p-RKIP dans un échantillon biologique, comprenant les étapes consistant à :
a) coopérer sur deux supports distincts:
a1) un anticorps reconnaissant une première séquence d'acides aminés présente dans la protéine p-RKIP et
a2) un anticorps reconnaissant une première séquence d'acides aminés présente dans la protéine RKIP;
b) ajouter une solution de blocage aux deux supports,
c) ajouter ledit échantillon biologique;
d) ajouter, dans les supports respectifs
d1) un anticorps reconnaissant une deuxième séquence d'acides aminés présente dans la protéine p-RKIP et
d2) un anticorps reconnaissant une deuxième séquence d'acides aminés présente dans la protéine RKIP ;
l'une desdites première et deuxième séquences d'acides aminés étant commune entre RKIP et p-RKIP, et l'autre étant une séquence contenant la phosphorylation de la sérine 153, et ainsi différente pour p-RKIP et RKIP;
e) ajouter un marqueur capable de déterminer une variation des propriétés optiques proportionnelle à la quantité dudit anticorps lié à ladite deuxième séquence d'acides aminés, lorsqu'il est en contact avec ledit anticorps, en reconnaissant ladite deuxième séquence d'acides aminés;
f) mesurer une propriété optique des deux échantillons et la comparer à une courbe d'étalonnage obtenue en mesurant la même propriété optique de deux séries de supports, dans lesquelles ont été ajoutés, dans l'ordre:
i) un anticorps reconnaissant ladite première séquence d'acides aminés présente dans la protéine p-RKIP pour la première de ladite série et ladite première séquence d'acides aminés présente dans la protéine RKIP pour la deuxième de ladite série
ii) une solution de blocage
iii) des quantités connues et croissantes d'un standard hybride recombinant comprenant à la fois lesdites premières et deuxièmes séquences d'acides aminés présentes dans la protéine p-RKIP pour la première de ladite série et à la fois lesdites premières et deuxièmes séquences d'acides aminés présentes dans la protéine RKIP pour la deuxième de ladite série;
iv) des anticorps reconnaissant spécifiquement ladite deuxième séquence d'acides aminés présente dans p-RKIP ou ladite deuxième séquence présente dans RKIP, respectivement;
v) un marqueur capable de déterminer une variation des propriétés optiques proportionnelle à la quantité dudit anticorps lié à ladite deuxième séquence d'acides aminés, lorsqu'il est en contact avec ledit anticorps, reconnaissant ladite deuxième séquence d'acides aminés.

2. Procédé selon la revendication 1, **caractérisé en ce que** ledit standard hybride recombinant qui comprend à la fois lesdites premières et deuxièmes séquences d'acides aminés présentes dans la protéine p-RKIP présente la séquence d'acides aminés rapportée dans ce qui suit:
DPDAPSRKDPKYRE-(linker) -GDHRGKFKVApSFRKKYELRA

3. Procédé selon la revendication 1 ou 2, **caractérisé en ce que** ledit standard hybride recombinant qui comprend à la fois lesdites premières et deuxièmes séquences d'acides aminés présentes dans la protéine RKIP présente la séquence d'acides aminés rapportée dans ce qui suit:
DPDAPSRKDPKYRE- (linker) -GDHRGKFKVASFRKKYELRA

4. Procédé selon la revendication 3, **caractérisé en ce que** ladite première séquence présente dans RKIP et ladite première séquence présente dans p-RKIP sont égales à la séquence rapportée dans ce qui suit: DPDAPSRKDPKYRE.

5. Procédé selon la revendication 4, **caractérisé en ce que** ladite deuxième séquence présente dans RKIP est la suivante:
GDHRGKFKVASFRKKYELRA
tandis que ladite deuxième séquence présente dans p-RKIP est la suivante:
GDHRGKFKVApSFRKKYELRA

6. Procédé selon l'une quelconque des revendications précédentes, **caractérisé en ce que** lesdits supports sont des puits d'une plaque de microtitration ELISA, **en ce que** l'on introduit après le point d) l'étape de :
d3) ajouter dans les supports respectifs des anticorps conjugués HRP spécifiques pour desdits anticorps qui reconnaissent lesdites deuxièmes séquences d'acides aminés,
et après le point iv) à ladite série de puits, des anticorps conjugués HRP spécifiques pour des anticorps qui reconnaissent lesdites deuxièmes séquences d'acides aminés sont ajoutés,
et **en ce que** ledit marqueur capable de déterminer une variation de propriétés optiques lorsqu'il est en contact avec ledit anticorps, reconnaisse que ladite deuxième séquence d'acides aminés est TMB et ladite propriété optique est la densité optique.

7. Procédé selon l'une quelconque des revendications précédentes, **caractérisé en ce que** lesdits supports sont des cartouches chromatographiques,
**en ce que** ledit échantillon biologique est ajouté à une extrémité de la cartouche,
**en ce que** lesdits anticorps qui reconnaissent lesdites premières séquences d'acides aminés sont activés dans une région de ladite cartouche distale par rapport au papier absorbant,
et **en ce que** lesdits anticorps qui reconnaissent lesdites deuxièmes séquences d'acides aminés adhèrent à la surface chromatographique de ladite cartouche dans une région proche du papier absorbant,
et **en ce que** ledit marqueur capable de déterminer une variation de propriétés optiques lorsqu'il est en contact avec ledit anticorps reconnaissant ladite deuxième séquence d'acides aminés est une sonde fluorescente et ladite propriété optique est une fluorescence à une longueur d'onde spécifique.

8. Procédé selon l'une quelconque des revendications précédentes, **caractérisé en ce que** ledit échantillon biologique est l'un parmi l'urine, le sérum, le plasma, la salive, le liquide brocho-alvéolaire, le liquide céphalo-rachidien, les selles, le sperme, un extrait de protéine cellulaire ou tissulaire ou un milieu de culture cellulaire.

9. Procédé de diagnostic pour le diagnostic du carcinome à cellules claires des cellules rénales, comprenant les étapes consistant à:
- mesurer la quantité de p-RKIP dans un échantillon d'urine avec le procédé selon l'une quelconque des revendications précédentes;
- en cas d'absence de p-RKIP dans l'échantillon d'urine, effectuer le diagnostic d'un carcinome à cellules claires des cellules rénales.

10. Procèdé de pronostic pour anticiper le développement d'un carcinome à cellules claires des cellules rénales, comprenant les étapes suivantes consistant à:
- mesurer la quantité de RKIP dans un échantillon d'urine avec le procédé selon l'une quelconque des revendications 1 à 6 ;
- si la quantité RKIP est supérieure à une valeur seuil, un pronostic favorable est déduit;
- si la quantité RKIP mesurée est inférieure à une valeur seuil, un pronostic non favorable est déduit.


### SEQUENCE LISTING

<110> FLUIDIA S.r.l.
<120> Method for quantitative assessment in biological sample of the
   phosphorylated and non-phosphorylated form of RKIP.
<130> 1306
<160> 5
<170> BiSSAP 1.3.6
<210> 1
   <211> 14
   <212> PRT
   <213> Artificial Sequence
<220>
   <223> Regione comune RKIP - pRKIP
<400> 1
   Asp Pro Asp Ala Pro Ser Arg Lys Asp Pro Lys Tyr Arg Glu
   1               5                   10
<210> 2
   <211> 14
   <212> PRT
   <213> Artificial Sequence
<220>
   <223> Standard Ricombinante ibrido RKIP - parte 1
<400> 2
   Asp Pro Asp Ala Pro Ser Arg Lys Asp Pro Lys Tyr Arg Glu
   1               5                   10
<210> 3
   <211> 20
   <212> PRT
   <213> Artificial Sequence
<220>
   <223> Standard ricombinante ibrido RKIP - parte 2
<400> 3
   Gly Asp His Arg Gly Lys Phe Lys Val Ala Ser Phe Arg Lys Lys Tyr
   1               5                   10                  15
   Glu Leu Arg Ala
               20
<210> 4
   <211> 14
   <212> PRT
   <213> Artificial Sequence
<220>
   <223> Standard ricombinante ibrido p-RKIP - parte 1
<400> 4
   Asp Pro Asp Ala Pro Ser Arg Lys Asp Pro Lys Tyr Arg Glu
   1               5                   10
<210> 5
   <211> 20
   <212> PRT
   <213> Artificial Sequence
<220>
   <223> Standard ricombinante ibrido p-RKIP - parte 2
<220>
   <221> MOD_RES
   <222> 11
   <223> PHOSPHORYLATION
<400> 5
   Gly Asp His Arg Gly Lys Phe Lys Val Ala Ser Phe Arg Lys Lys Tyr
   1               5                   10                  15
   Glu Leu Arg Ala
               20